# EUROPEAN PATENT SPECIFICATION

(11) **EP 3 512 446 B1**
(45) Date of publication and mention of the grant of the patent: **20.11.2019**
(21) Application number: 17764850.8
(22) Date of filing: 15.09.2017
(51) Int. Cl.: A61B 18/20, A61B 18/00

(54) **A CUTTING ELEMENT FOR A HAIR CUTTING DEVICE**
SCHNEIDEELEMENT FÜR EINE HAARSCHNEIDEVORRICHTUNG
ÉLÉMENT DE COUPE POUR UN DISPOSITIF DE COUPE DE CHEVEUX

(30) Priority: 16.09.2016 EP 16189358
(43) Date of publication of application: 24.07.2019
(73) Proprietor: Koninklijke Philips N.V., 5656 AE Eindhoven (NL)
(72) Inventor: THUMMA, Kiran Kumar, 5656 AE Eindhoven (NL); JOHNSON, Mark Thomas, 5656 AE Eindhoven (NL); VERHAGEN, Rieko, 5656 AE Eindhoven (NL); BOURQUIN, Yannyk Parulian Julian, 5656 AE Eindhoven (NL); MOESKOPS, Bastiaan Wilhelmus Maria, 5656 AE Eindhoven (NL)
(74) Representative: Uittenbroek, Arie Leendert
(86) International application number: PCT/EP2017/073321
(87) International publication number: WO 2018/050843

(56) References cited:
- WO-A1-2014/143670
- GB-A- 2 430 027
- US-A1- 2004 067 035
- US-A1- 2008 244 912
- US-A1- 2014 140 091

## Description

### FIELD OF THE INVENTION

The invention relates to a cutting element for use with a hair cutting device for cutting (e.g. shaving) hair on a body of a subject and, in particular, a cutting element which includes optical structures. The invention also relates to a hair cutting device comprising such a cutting element.

### BACKGROUND OF THE INVENTION

Shaving devices for cutting or shaving hair on a body of a subject typically make use of one or more blades that cut hairs as the device is moved across the skin of the subject. The blades can be static within the device, for example as in a wet razor, whereas in other types of devices, for example electric shavers, one or more blade elements can be actuated (e.g. rotated or oscillated) in order to produce a cutting action.

However, an alternative type of shaving device has been proposed in WO 2014/143670 that makes use of laser light. In particular, a laser light source is provided that is configured to generate laser light having a wavelength selected to target a predetermined chromophore to effectively cut a hair shaft. A fibre optic is located on a shaving portion of the device that is positioned to receive the laser light from the laser light source at a proximal end, conduct the laser light from the proximal end toward a distal end, and emit the light out of a cutting region of the fibre optic and toward hair when the cutting region is brought in contact with the hair.

### SUMMARY OF THE INVENTION

To achieve good shaving closeness, the cutting element of the shaving device (e.g. the fibre optic in the case of WO 2014/143670), needs to be brought very close to the skin or even touch the skin. However, there exists a possibility that light propagating through the fibre optic will couple into the skin or, more particularly, into defects or lesions on the skin, such as moles. This will potentially lead to burning or irritation of the skin and create a significant safety issue for this type of shaving device.

Thus, to reduce the risk of damage or injury to the skin of the subject from optical energy, it is proposed herein to provide optical structures within the cutting element to enable the cutting element to function as a sensor for detecting the presence of an object that comes into contact with the cutting element.

According to a first aspect, a cutting element for use in a hair cutting device comprises an optical waveguide having a sidewall, wherein a portion of the sidewall forms a cutting face for contacting hair. The optical waveguide includes a plurality of optical structures spaced along its length, the optical structures being configured to reflect light at one or more wavelengths. Such an arrangement enables the optical waveguide to be used both for cutting hair and as a sensor to detect one or more objects coming into contact with the waveguide.

The optical waveguide is such that, when broadband light is directed along the optical waveguide: if the cutting face of the optical waveguide is not in contact with a target object, then each of the plurality of optical structures reflects light having a first wavelength; and if the cutting face of the optical waveguide is in contact with a target object, then at least one of the optical structures reflects light having a second wavelength different to the first wavelength. The terms "object" and "target object" used herein is intended to mean an object whose presence is intended to be detected, for example a hair, skin, or a skin lesion such as a mole or a skin tag.

In some embodiments, each of the plurality of optical structures may comprise a grating and, in some embodiments, each optical structure may comprise a fibre Bragg grating or a photonic crystal.

The optical structures may, in some embodiments, all be configured to reflect light having the same wavelength. In other embodiment, however, each of the optical structures may be configured to reflect light having a unique wavelength. With each optical structure being configured to reflect light having a unique wavelength, it may be possible to determine which optical structure reflect has reflected the detected light as each structure corresponds to a representative frequency or wavelength of light.

The optical waveguide may be such that, when light having a specific wavelength is directed along the optical waveguide, some of the light having the specific wavelength is able to couple out from the cutting face of the optical waveguide into hair to initiate cutting or melting of the hair.

According to a second aspect, a hair cutting device for cutting hair on a body of a subject comprises a first light source for generating laser light at one or more specific wavelengths corresponding to wavelengths absorbed by one or more chromophores in hair; a second light source for generating broadband light; and a cutting element coupled to the first light source to receive laser light and to the second light source to receive broadband light. The cutting element may comprise a cutting element as described above.

The hair cutting device may further comprise a detector for detecting light reflected from one or more of the plurality of optical structures.

In some embodiments, the hair cutting device may further comprise a processor configured to determine whether any of the light reflected from one or more of the plurality of optical structures has a wavelength different from a defined wavelength; and, upon determining that the wavelength of the reflected light is different from a defined wavelength, generate an instruction signal. The processor may be configured to determine, based on the wavelength of the reflected light, the nature of an object likely to have come into contact with the optical waveguide, causing the one or more optical structures to reflect light at the wavelength different to the defined wavelength.

The processor may be further configured to generate a first instruction signal when the determined nature of the object indicates that the object is a hair, wherein the first instruction signal comprises an instruction to the first light source to generate relatively high-powered laser light. In some embodiments, the processor may be configured to generate a second instruction signal when the determined nature of the object indicates that the object is skin or a skin lesion, wherein the second instruction signal comprises an instruction to the first light source to generate relatively low-powered laser light.

According to a third aspect, a method of operating a hair cutting device is defined. The hair cutting device comprises a first light source for generating laser light, a second light source for generating broadband light, and a cutting element coupled to the first light source and the second light source. The cutting element may comprise an optical waveguide having a plurality of optical structures spaced along its length. The method may comprise: delivering broadband light along the optical waveguide; detecting a response from broadband light reflected by each of the plurality of optical structures, each response having a representative frequency; and, upon detection of a particular response having a particular frequency different to the representative frequency, generating an instruction signal.

In some embodiments, the method may further comprise determining, from the particular response, the nature of an object likely to have contacted the optical waveguide to cause the response of the particular frequency. If it is determined that the object likely to have contacted the optical waveguide is a hair, then the generated instruction signal may comprise an instruction to the first light source to generate relatively high powered laser light. If it is determined that the object likely to have contacted the optical waveguide is skin or a skin lesion, then the generated instruction signal may comprise an instruction to the first light source to generate relatively low powered laser light.

Other advantageous features will become apparent from the following description.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a block diagram of a hair cutting device according to an embodiment of the invention;
Fig. 2 is a pair of schematic drawings showing different views of an exemplary hair cutting device according to an embodiment of the invention;
Fig. 3 is a graph illustrating the refractive index of hair;
Fig. 4 is an illustration of an optical waveguide cutting element according to a specific embodiment of the invention; and
Figs. 5A and 5B are perspective drawings of an optical waveguide including optical structures.

### DETAILED DESCRIPTION OF THE EMBODIMENTS

As noted above, the present invention provides an improvement in the cutting ability and safety of a laser light-based shaving device, for example as described in WO 2014/143670. In particular, it has been recognised that by detecting the presence of an object coming into contact with the device, suitable measures can be taken depending on the nature of the detected object. Thus, the risk of pain or irritation of the skin can be reduced.

It will be appreciated that the invention is applicable to shaving devices (e.g. razors or electric shavers), and any other type of device that is used to cut hair (e.g. hair clippers), even if those devices do not necessary aim to provide a 'clean shave' (i.e. to remove hair at the level of the skin).

The invention is described with reference to light having a particular "wavelength" and/or "frequency". Since the wavelength of light is inversely proportional to the frequency of light, those terms are used interchangeably in this description.

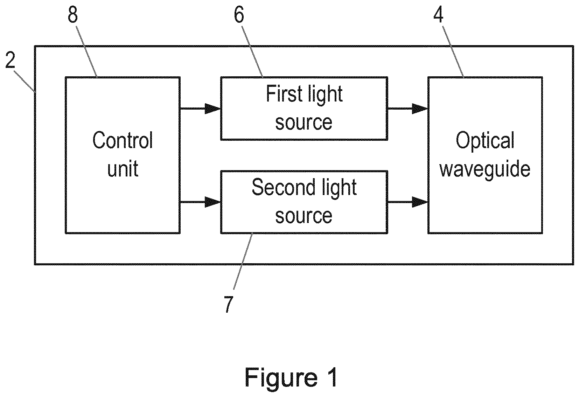

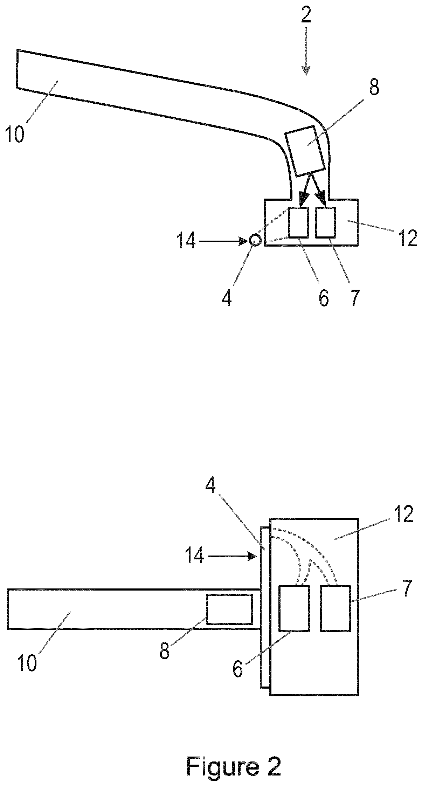

Fig. 1 is a block diagram of a hair cutting device 2 according to an embodiment of the invention. Fig. 2 shows a hair cutting device 2 in the form of a handheld razor according to an exemplary embodiment of the invention. The hair cutting device 2 is for cutting (e.g. shaving) hair on a body of a subject. The subject may be a person or an animal. The hair may be facial hair (i.e. hair on the subject's face), or hair on the subject's head or other part of their body (legs, chest, etc.).

The hair cutting device 2 comprises a cutting element 4 that enables hair to be cut as the hair cutting device 2 is moved over the skin of a subject. The cutting element 4 is an optical waveguide 4 that is arranged on the hair cutting device 2 so that the optical axis of the optical waveguide 4 (i.e. the line along which light typically propagates through the optical waveguide 4) is generally perpendicular to the direction in which the hair cutting device 2 is moved so that hairs contact the sidewall of the optical waveguide 4 (the sidewall corresponding to the long edge of the optical waveguide 4) as the hair cutting device 2 is moved across the skin of the subject. In some embodiments, the optical waveguide 4 is an optical fibre, although those skilled in the art will be aware of other types of optical waveguide that can be used according to the invention, such as a slab waveguide, a strip waveguide or a photonic crystal waveguide. An optical fibre comprises a core, and in some embodiments also comprises a cladding, which may or may not fully encompass the core (e.g. part of the core may be exposed).

A first light source 6 is provided in the hair cutting device 2 that generates laser light at one or more specific wavelengths, and second light source 7 is provided in the hair cutting device 2 that generates broadband light. In some embodiments, the first and second light sources 6, 7 may form parts of a single, combined light source capable of generating laser light and broadband light. The first and second light sources 6, 7 are optically coupled to the optical waveguide 4. The laser light generated by the light source 6 is coupled into the optical waveguide 4 (and specifically coupled into an end of the optical waveguide 4 so that the laser light propagates through the optical waveguide 4).

The first light source 6 is configured to generate laser light at one or more specific wavelengths that can be used to cut or burn through hair. In particular, each wavelength corresponds to the wavelength of light absorbed by a chromophore that is found in hair. As is known, a chromophore is the part of a molecule that provides the molecule with its colour. Thus, the laser light will be absorbed by the chromophore and converted into heat which will melt or burn the hair or otherwise destroy the bonds in the molecules of the hair, and it is this melting or burning that provides the cutting action of the hair cutting device 2.

Suitable chromophores that can be targeted by the laser light generated by the first light source 6 include, but are not limited to, melanin, keratin and water. Suitable wavelengths of laser light that can be used include, but are not limited to, wavelengths selected from the range 380 nm (nanometres) to 500 nm and 2500 nm to 3500 nm. Those skilled in the art will be aware of the wavelengths of light that are absorbed by these chromophores, and thus also the specific wavelengths of light that the first light source 6 should generate for this purpose, and further details are not provided herein.

In some embodiments the first light source 6 can be configured to generate laser light at a plurality of wavelengths (either simultaneously or sequentially), with each wavelength being selected to target a different type of chromophore. This can improve the cutting action of the optical waveguide 4 since multiple types of molecules in the hair may be burnt using the laser light. Alternatively multiple light sources can be provided that each generate laser light at a respective wavelength, and each light source can be coupled to a respective optical waveguide 4 to provide multiple cutting elements 4 in the device 2.

The second light source 7 is configured to generate broadband, or white, light having a range of wavelengths which may or may not fall within the visible spectrum.

The hair cutting device 2 also comprises a control unit 8 that controls the operation of the hair cutting device 2, and in particular is connected to the first and second light sources 6, 7 to control the activation and deactivation of the light sources 6, 7 (and in some embodiments control the wavelength and/or intensity of the light generated by the light sources 6, 7). The control unit 8 may activate and deactivate one or both of the light sources 6, 7 in response to an input from a user of the hair cutting device 2. The control unit 8 can comprise one or more processors, processing units, multi-core processors or modules that are configured or programmed to control the hair cutting device 2.

As noted above, Fig. 2 shows a hair cutting device 2 that is in the form of a handheld wet razor. Fig. 2 shows a side view and a bottom view of the razor 2. The razor 2 comprises a handle 10 for the subject (or other user of the device 2) to hold, and a head portion 12 that includes the cutting element 4 (optical waveguide/fibre). As shown, the optical waveguide 4 is arranged along an edge of the head portion, and a part of the optical waveguide 4 forms (or corresponds to) a cutting face 14. The cutting face 14 is the part of the optical waveguide 4 that is intended to come into contact with hair as the hair cutting device 2 is moved across the skin of the subject. The first and second light sources 6, 7 and the control unit 8 are shown as being incorporated into the head portion 12 and handle 10 respectively, but it will be appreciated that the positions of these components in the hair cutting device 2 as shown in Fig. 2 is not limiting. Likewise it will be appreciated that the embodiment shown in Fig. 2 is merely an example, and the invention can be incorporated or used in any type of hair cutting device 2 that comprises an optical waveguide cutting element 4 as described herein.

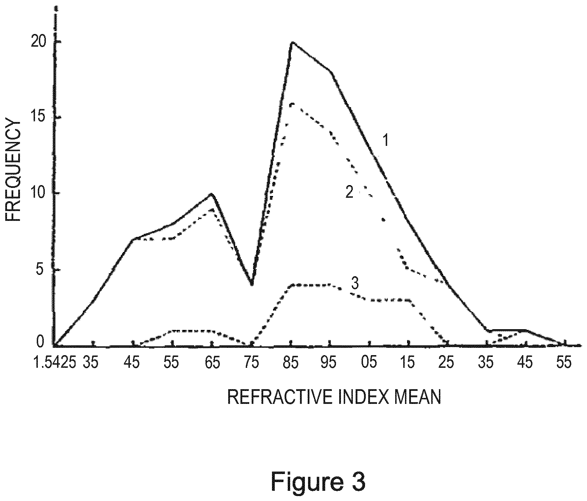

The graph in Fig. 3 illustrates the refractive index of hair, which can be found in a paper by M. D. Greenwell, A. Willner, Paul L. Kirk: Human Hair Studies: III. Refractive Index of Crown Hair, 31 Am. Inst. Crim. L. & Criminology 746 (1940-1941). Curve 1 is a composite line, curve 2 is a line representing the refractive index for Caucasian people, and curve 3 is a line representing the refractive index for non-Caucasian people. Thus, it can be seen that the refractive index of hair is between (approximately) 1.545 and 1.555, although there will be variation between individuals. For example the above paper also recognises that the refractive index of hair can depend on the sex of the subject, e.g. the refractive index of hair on a female is generally higher than the refractive index of hair on a male.

As is known, the optical waveguide 4 acts as a waveguide for the light coupled from the light sources 6, 7 through the occurrence of total internal reflection, since the refractive index of air is lower than that of the optical waveguide 4. However, if an object that has a refractive index higher than the optical waveguide 4 is put into contact with the optical waveguide 4, then the total internal reflection is 'frustrated' and light can couple from the optical waveguide 4 into that object. Thus, in order for light to be coupled into a hair from the optical waveguide 4 (to provide the cutting action according to the invention), the optical waveguide 4 must have the same or a lower refractive index than hair at the point at which the hair contacts the optical waveguide 4. Thus, the optical waveguide 4 must have the same or a lower refractive index than hair at least at the cutting face 14 portion of the optical waveguide 4. Preferably the refractive index of the optical waveguide 4 at the cutting face 14 is the same as that of hair since that provides the best coupling of light from the optical waveguide 4 to the hair.

Thus, in some embodiments, the refractive index of the optical waveguide 4 at least at the cutting face 14 is equal to or lower than 1.56. More preferably the refractive index of the optical waveguide 4 at least at the cutting face 14 is equal to or lower than 1.55. Even more preferably, the refractive index of the optical waveguide 14 at least at the cutting face 14 is equal to or lower than 1.54, since this refractive index is below the refractive indices identified in Fig. 3.

In some embodiments, a lower bound for the refractive index of the optical waveguide 4 at the cutting face 14 can be 1.48, 1.51, 1.53 or 1.54.

A range of values from which the refractive index of the optical waveguide 4 is selected can be formed from any combination of the upper and lower refractive index bounds set out in the preceding paragraphs.

The optical waveguide/fibre 4 can be made from any suitable material or combination of materials. For example optical waveguides/fibres can be composed of or comprise silica, fluoride glass, phosphate glass, chalcogenide glass, and/or crown glass (such as BK7).

As noted above, the hair cutting device 2 includes a first light source 6 for generating laser light and a second light source 7 for generating broadband light. When laser light propagates through the optical waveguide 4, some of the laser light is caused to couple out from the optical waveguide and into the surrounding medium, such as hair. As such, the laser light is used for cutting hair brought into contact with the cutting element 4.

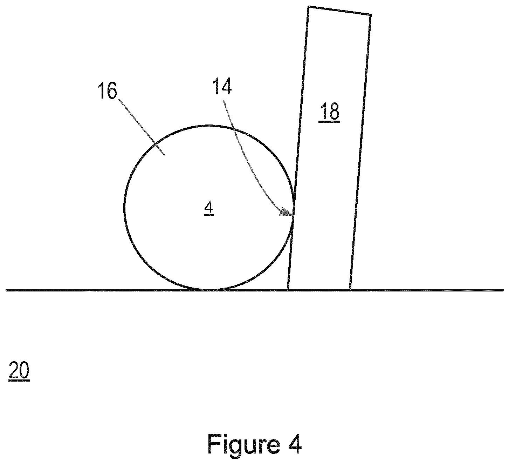

Fig. 4 illustrates an exemplary embodiment of the cutting element 4 (optical waveguide 4) according to the invention. In Fig. 4, only a portion of the optical waveguide 4 part of the hair cutting device 2 is shown, and the optical waveguide 4 is shown in perspective view. No support structure for the optical waveguide 4 is shown.

In Fig. 4, an optical waveguide 4 is shown that has a core 16. In this illustrated embodiment, the optical waveguide 4 does not include any cladding around the core 16. However, it will be appreciated that in some embodiments the optical waveguide 4 can comprise cladding around the core 16, although preferably no cladding is present along the cutting face 14 (and indeed, in some embodiments the cutting face 14 can correspond to those parts of the optical waveguide 4 where there is no cladding).

The optical waveguide 4 is shown in contact with a hair 18 and the skin 20. The portion of the sidewall of the core 16/optical waveguide 4 that is intended to contact hairs during use forms the cutting face 14. As described above, the refractive index of the core 16 is the same or lower than the refractive index of hair.

The core 16 may have a uniform refractive index (i.e. the same refractive index throughout the core 16), or it may be a graded index fibre, which means that the refractive index decreases with increasing distance from the optical axis.

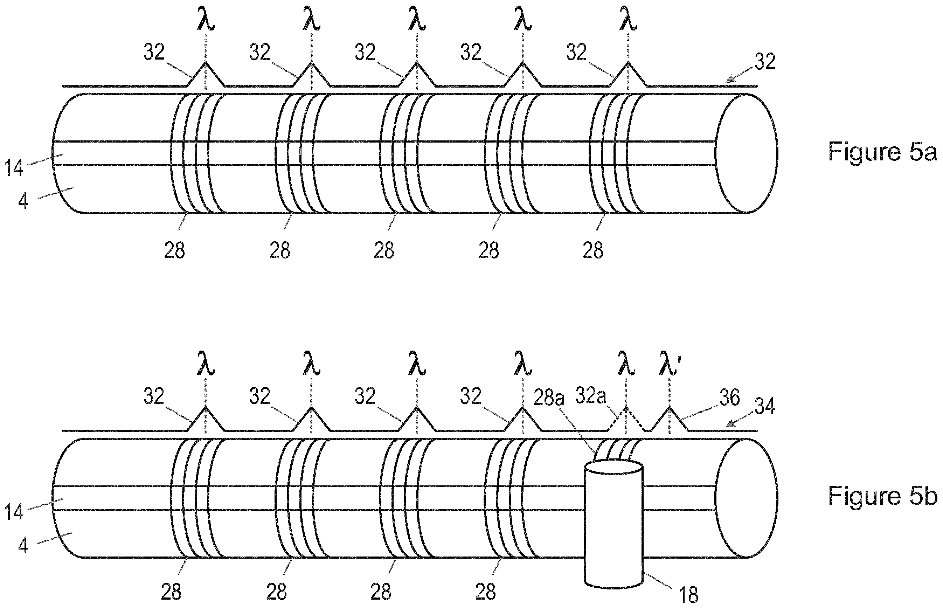

In addition to cutting hairs 18 by coupling laser light out from the optical waveguide 4, the optical waveguide described herein can also be used as a sensor, as described below with reference to Fig. 5.

Figs. 5A and 5B illustrate a portion of the optical waveguide 4 of a cutting device 2, with the cutting face 14 extending along the length of the optical waveguide. In Fig. 5A, the cutting face 14 of the optical waveguide 4 is not in contact with a hair. However, in Fig. 5B, the cutting face 14 of the optical waveguide 4 is in contact with a hair 18. In the embodiment shown in Fig. 5, the optical waveguide 4 is an optical fibre which is shown as having a core with no cladding. A sensing, or detecting, function may be performed using a plurality of optical structures 28 spaced along the length of the optical waveguide 4, which are configured to reflect light at one or more wavelengths. In some embodiments, the optical structures 28 are spaced evenly, while in other embodiments, there may be larger or smaller spaces between adjacent optical structures. Fig. 5 shows five optical structures spaced along the length of the optical waveguide 4. However, in other embodiments, more or fewer optical structures 28 may be included.

Each optical structure 28 is configured to reflect light having a particular wavelength, such that some of the broadband light propagating along the optical waveguide 4 is reflected from each of the optical structures back along the waveguide. The optical structures 28 may be any structure suitable for being incorporated into, coupled to, or otherwise added to an optical waveguide, and suitable for reflecting light at a particular wavelength. The optical structures 28 may, therefore, be a grating or a photonic crystal. In some embodiments in which the optical waveguide 4 is an optical fibre, each optical structure 28 may be a fibre Bragg grating, which is a type of distributed Bragg reflector capable of reflecting light having a particular wavelength, and transmitting light at all other wavelengths. Fibre Bragg gratings may be formed in optical fibres using a laser, such as an ultraviolet laser, to inscribe a controlled variation in refractive index in the core of the fibre. In general, the optical structures 28 are structures capable of creating a variation in the refractive index within the optical waveguide 4.

According to some embodiments of the invention, therefore, the optical waveguide may be such that, when broadband light is directed along the optical waveguide, if the cutting face of the optical waveguide is not in contact with a target object, then each of the plurality of optical structures reflects light having a first wavelength; and if the cutting face of the optical waveguide is in contact with a target object, then at least one of the optical structures reflects light having a second wavelength different to the first wavelength. Here, a target object refers to an object that might come into contact with the optical waveguide during use, such as a hair, skin, or a skin lesion.

In order to detect light reflected by one or more of the optical structures, the cutting device 2 may include a detector (not shown), which may be located within the head portion 12 or the handle 10, for detecting light reflected from one or more of the plurality of optical structures. The detector may be associated with and/or connected to the control unit 8 and/or to one or more other processing apparatus for analysing signals received by the detector. The detector (and associated processing apparatus) may be configured to receive light reflected by one or more of the optical structures 28 and determine the frequency or wavelength of the reflected light.

In the example discussed with reference to Fig. 5, each of the optical structures 28 is a fibre Bragg grating, and each grating is configured to reflect light having the same particular wavelength. An example response signal 30 is shown in Fig. 5A above the optical waveguide 4. The response signal 30 includes a peak 32 associated with each grating 28, which is indicative of the detection of a reflection of light at a particular wavelength λ from each grating.

If the optical waveguide 4 comes into contact with an object, such as a hair or a skin lesion (for example, a skin tag or a mole), the object applies a force onto the optical waveguide 4. If sufficient force is applied to the optical waveguide 4, then one or more of the gratings 28 within the waveguide will be put under stress and be caused to stretch or otherwise deform. A deformed or stretched grating will reflect light having a wavelength (or frequency) different to the wavelength (or frequency) of the light reflected by a grating which has not been stretched or deformed. Therefore, if a detection (for example, by the detector) is made of reflected light having a wavelength different to the wavelength at which the gratings 28 are configured to reflect, then it can be deduced that the optical waveguide 4 has come into contact with an object. Objects of different sizes and/or materials may apply different forces on the optical waveguide and, consequently, may cause the gratings 28 to deform by different amounts. Accordingly, the shift in frequency or wavelength of light reflected by a grating 28 depends on the degree by which the grating is deformed. It is therefore possible to determine the nature of an object in contact with the optical waveguide 4 by the frequency or wavelength of light reflected by a particular grating 28 or by the shift in the frequency of reflected light from the frequency of light expected to be reflected.

In Fig. 5B, the optical waveguide 4 is in contact with the hair 18 at a location along the optical waveguide corresponding to a grating labelled 28a. The hair 18 causes the optical waveguide 4 and, specifically, the grating 28a to deform and, in its deformed state, the grating 28a reflects light at a wavelength different from the wavelength of light reflected by the grating when it is not in contact with the hair. An example response signal 34 is shown in Fig. 5B above the optical waveguide 4. As with Fig. 5A, the signal 34 includes peaks 32 which correspond to the gratings 28 which are not deformed by the hair 18, or may be deformed by a small, even negligible amount. In addition, however, the signal 34 also includes a peak 36 (having a wavelength λ') which is shifted relative to a peak (labelled 32a) that would have been expected from the grating 28a reflecting light when in an un-deformed state.

As noted above, it is possible to determine, from the shift in frequency of the reflected light resulting from the force on the grating 28, the nature of the object contacting the optical waveguide 4. In some embodiments, different action is taken in response to detecting contact with different objects. Since the sensing function of the optical waveguide 4 (i.e. the detection of an object in contact with the waveguide) uses broadband light, rather than the laser light used for the hair cutting function, the cutting device 2 may be configured to vary the power of the laser light generated by the first light source 6 depending on the nature of an object in contact with the optical waveguide 4. In some embodiments, a signal may be generated in response to detecting a frequency shift in the reflected light and determining that the frequency shift is the result of an object coming into contact with the optical waveguide 4.

The processing apparatus associated with the detector (e.g. the control unit 8) may be configured to determine whether any of the light reflected from one or more of the plurality of optical structures has a wavelength different from a defined wavelength; and, upon determining that the wavelength of the reflected light is different from a defined wavelength, generate an instruction signal.

The signal may be an instruction signal to control the power of laser light generated by the first light source 6. For example, if it is determined that the object likely to have contacted the optical waveguide is a hair, then a first instruction signal may be generated, the first instruction signal including an instruction to the first light source 6 to generate relatively high-powered laser light. In some embodiments the first instruction signal may include an instruction to the first light source 6 to increase the power of the laser light generated. High-powered laser light, or laser light having an increased power, may assist in initiating the melting or cutting of the hair. If it is determined that the object likely to have contacted the optical waveguide is skin or a skin lesion, then a second instruction signal may be generated, the second instruction signal including an instruction to the first light source 6 to generate relatively low-powered laser light. In some embodiments the second instruction signal may include an instruction to the first light source 6 to reduce the power of the laser light generated, or even stop the light source from generating laser light altogether. Low-powered laser light, or laser light having a reduced power, may lower the likelihood that the skin or skin lesion in contact with the optical waveguide 4 will be burnt or damaged by laser light coupling out from the optical waveguide.

In some embodiments, the power of the laser light generated by the first light source may be selected based on the number of objects in contact with the optical waveguide at a particular time. For example, if it is determined that a first hair is likely to have contacted the optical waveguide, then the first light source 6 may be configured, or instructed, to generate laser light at a first power. If it is determined that a second hair is likely to have contacted the optical waveguide while the optical waveguide is in contact with the first hair, then the first light source 6 may be configured, or instructed, to generate laser light at a second higher power. Thus, the power of the laser light may be increased as more hairs come into contact with the optical waveguide, to ensure that the cutting device 2 is able to cut through all of the hairs. Similarly, as hairs are cut, and are no longer in contact with the waveguide 4, the power of the laser light may be reduced or set to a third, lower power.

As the optical waveguide 4 is moved over skin by a user, it is likely to encounter numerous minor bumps and protrusions which might supply a force onto a portion of the optical waveguide. To avoid a false indication that contact has been made with a skin lesion, such as a mole, the detector (and/or associated processing apparatus) may be configured to ignore shifts in the wavelength (or frequency) of reflected light below a defined threshold. For example, if a minor skin defect (e.g. a protrusion caused by an uneven skin surface) contacts the optical waveguide and applies a small force, sufficient to deform one of the optical structures 28, then a small but detectable shift in the wavelength of reflected light may occur. However, if the wavelength shift falls below a defined threshold, then it may not be desirable or necessary to decrease the power of the laser light generated by the first light source 6 and, therefore, the laser light power may not be changed. In case the wavelength shift is above the defined threshold the power of the laser light generated by the first laser source 6 may be decreased. In other words, when slight contact with skin is detected, thus a wavelength shift below a defined threshold, the power of the laser light may remain unchanged as this slight contact is expected not to result in significant damage..However when firm contact with the skin is detected, thus a wavelength shift above the defined threshold, it is more likely that skin damage will occur and the power of the laser light is decreased to prevent such damage. In some embodiments, the defined threshold may be plus or minus 5 nanometres.

With the embodiments described so far, contact of an object with the optical waveguide anywhere along its length will be detected (as long as the resulting wavelength/frequency shift is above a defined threshold), but it may not be possible to determine the exact location of the detected object along the length of the optical waveguide. In other words, if all of the optical structures 28 are configured to reflect light at the same wavelength, then it may not be possible (or at least easy) to determine which optical structure caused the frequency-shifted light to be reflected.

In some embodiments, each optical structure along the length of the optical waveguide may be configured to reflect light having a unique wavelength (or frequency) or a unique range of wavelengths (or frequencies). In this way, the unique wavelength or range of wavelengths of light expected to be reflected by each optical structure 28 is known, so if light having a wavelength different to any of the expected wavelengths is detected, then it is possible to determine which of the optical structures caused the wavelength-shifted light to be reflected and, therefore, the position along the optical waveguide 4 at which contact with an object has been made.

As discussed herein, a cutting element 4 of a hair cutting device 2 may be used to cut hair, using laser light, and sensor objects coming into contact with the cutting element, using broadband light. Aspects of the invention therefore relate to the cutting element, and a hair cutting device including the cutting element. A further aspect of the invention relates to a method of operating a hair cutting device, the hair cutting device comprising a first light source for generating laser light, a second light source for generating broadband light, and a cutting element coupled to the first light source and the second light source. The cutting element comprises an optical waveguide having a plurality of optical structures spaced along its length. The method comprises delivering broadband light along the optical waveguide; detecting a response from broadband light reflected by each of the plurality of optical structures, each response having a representative frequency; and, upon detection of a particular response having a particular frequency different to the representative frequency, generating an instruction signal. From the particular response, the method may the nature of an object likely to have contacted the optical waveguide to cause the response of the particular frequency, in the manner discussed above.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A cutting element for use in a hair cutting device, the cutting element comprising:
an optical waveguide having a sidewall, wherein a portion of the sidewall forms a cutting face for contacting hair, wherein the optical waveguide includes a plurality of optical structures spaced along its length, the optical structures being configured to reflect light at one or more wavelengths.
wherein the optical waveguide is such that, when broadband light is directed along the optical waveguide:
- if the cutting face of the optical waveguide is not in contact with a target object, then each of the plurality of optical structures reflects light having a first wavelength; and
- if the cutting face of the optical waveguide is in contact with a target object, then at least one of the optical structures reflects light having a second wavelength different to the first wavelength.

2. A cutting element according to claim 1, wherein each of the plurality of optical structures comprises a fibre Bragg grating or a photonic crystal.

3. A cutting element according to claim 1 or claim 2, wherein each of the optical structures is configured to reflect light having a unique wavelength.

4. A cutting element according to any of claims 1 to 3, wherein the optical waveguide is such that, when light having a specific wavelength is directed along the optical waveguide, some of the light having the specific wavelength is able to couple out from the cutting face of the optical waveguide into hair to initiate cutting or melting of the hair.

5. A hair cutting device for cutting hair on a body of a subject, the hair cutting device comprising:
a first light source for generating laser light at one or more specific wavelengths corresponding to wavelengths absorbed by one or more chromophores in hair;
a second light source for generating broadband light; and
a cutting element coupled to the first light source to receive laser light and to the second light source to receive broadband light, the cutting element comprising a cutting element according to any of claims 1 to 4.

6. A hair cutting device according to claim 5, further comprising:
a detector for detecting light reflected from one or more of the plurality of optical structures.

7. A hair cutting device according to claim 5 or claim 6, further comprising a processor configured to:
determine whether any of the light reflected from one or more of the plurality of optical structures has a wavelength different from a defined wavelength; and
upon determining that the wavelength of the reflected light is different from a defined wavelength, generate an instruction signal.

8. A hair cutting device according to claim 7, wherein the processor is configured to:
determine, based on the wavelength of the reflected light, the nature of an object likely to have come into contact with the optical waveguide, causing the one or more optical structures to reflect light at the wavelength different to the defined wavelength.

9. A hair cutting device according to claim 8, wherein the processor is configured to:
generate a first instruction signal when the determined nature of the object indicates that the object is a hair, wherein the first instruction signal comprises an instruction to the first light source to generate relatively high-powered laser light.

10. A hair cutting device according to claim 8 or claim 9, wherein the processor is configured to:
generate a second instruction signal when the determined nature of the object indicates that the object is skin or a skin lesion, wherein the second instruction signal comprises an instruction to the first light source to generate relatively low-powered laser light.

11. A method of operating a hair cutting device, the hair cutting device comprising a first light source for generating laser light, a second light source for generating broadband light, and a cutting element coupled to the first light source and the second light source, the cutting element comprising an optical waveguide having a plurality of optical structures spaced along its length, the method comprising:
delivering broadband light along the optical waveguide;
detecting a response from broadband light reflected by each of the plurality of optical structures, each response having a representative frequency; and
upon detection of a particular response having a particular frequency different to the representative frequency, generating an instruction signal.

12. A method according to claim 11, comprising:
determining, from the particular response, the nature of an object likely to have contacted the optical waveguide to cause the response of the particular frequency.

13. A method according to claim 12, wherein, if it is determined that the object likely to have contacted the optical waveguide is a hair, then the generated instruction signal comprises an instruction to the first light source to generate relatively high-powered laser light.

14. A method according to claim 12 or claim 13, wherein, if it is determined that the object likely to have contacted the optical waveguide is skin or a skin lesion, then the generated instruction signal comprises an instruction to the first light source to generate relatively low-powered laser light.

## Patentansprüche

1. Schneidelement zur Verwendung in einer Haarschneidevorrichtung, wobei das Schneidelement umfasst:
einen Lichtwellenleiter mit einer Seitenwand, wobei ein Abschnitt der Seitenwand eine Schneidfläche zum Inkontaktbringen mit Haar bildet, wobei der Lichtwellenleiter eine Vielzahl von optischen Strukturen beinhaltet, die entlang seiner Länge beabstandet sind, wobei die optischen Strukturen konfiguriert sind, um Licht bei einer oder mehreren Wellenlängen zu reflektieren.
wobei der Lichtwellenleiter so beschaffen ist, dass, wenn Breitbandlicht entlang des Lichtwellenleiters gerichtet wird:
- wenn die Schneidfläche des Lichtwellenleiters nicht in Kontakt mit einem Zielobjekt ist, dann reflektiert jede der Vielzahl von optischen Strukturen Licht mit einer ersten Wellenlänge; und
- wenn die Schneidfläche des Lichtwellenleiters in Kontakt mit einem Zielobjekt ist, dann reflektiert mindestens eine der optischen Strukturen Licht mit einer zweiten Wellenlänge, die sich von der ersten Wellenlänge unterscheidet.

2. Schneidelement nach Anspruch 1, wobei jede der Vielzahl von optischen Strukturen ein Faser-Bragg-Gitter oder einen photonischen Kristall umfasst.

3. Schneidelement nach Anspruch 1 oder Anspruch 2, wobei jede der optischen Strukturen konfiguriert ist, um Licht mit einer einzigartigen Wellenlänge zu reflektieren.

4. Schneidelement nach einem der Ansprüche 1 bis 3, wobei der Lichtwellenleiter so beschaffen ist, dass, wenn Licht mit einer bestimmten Wellenlänge entlang des Lichtwellenleiters gerichtet wird, ein Teil des Lichts mit der bestimmten Wellenlänge in der Lage ist, aus der Schneidfläche des Lichtwellenleiters heraus in das Haar hinein zu koppeln, um das Schneiden oder Schmelzen des Haares einzuleiten.

5. Haarschneidevorrichtung zum Schneiden von Haaren auf einem Körper eines Probanden, wobei die Haarschneidevorrichtung umfasst:
eine erste Lichtquelle zum Erzeugen von Laserlicht bei einer oder mehreren spezifischen Wellenlängen, die den Wellenlängen entsprechen, die von einem oder mehreren Chromophoren im Haar absorbiert werden;
eine zweite Lichtquelle zum Erzeugen von Breitbandlicht; und
ein Schneidelement, das mit der ersten Lichtquelle zum Empfangen von Laserlicht und mit der zweiten Lichtquelle zum Empfangen von Breitbandlicht gekoppelt ist, wobei das Schneidelement ein Schneidelement nach einem der Ansprüche 1 bis 4 umfasst.

6. Haarschneidevorrichtung nach Anspruch 5, weiter umfassend:
einen Detektor zum Erfassen von Licht, das von einer oder mehreren der Vielzahl von optischen Strukturen reflektiert wird.

7. Haarschneidevorrichtung nach Anspruch 5 oder Anspruch 6, weiter umfassend einen Prozessor, der konfiguriert ist, um:
zu bestimmen, ob das von einer oder mehreren der Vielzahl von optischen Strukturen reflektierte Licht eine Wellenlänge aufweist, die sich von einer definierten Wellenlänge unterscheidet; und
nach dem Bestimmen, dass sich die Wellenlänge des reflektierten Lichts von einer definierten Wellenlänge unterscheidet, ein Befehlssignal zu erzeugen.

8. Haarschneidevorrichtung nach Anspruch 7, wobei der Prozessor konfiguriert ist, um:
basierend auf der Wellenlänge des reflektierten Lichts die Beschaffenheit eines Objekts zu bestimmen, das wahrscheinlich mit dem Lichtwellenleiter in Kontakt gekommen ist, wodurch die eine oder die mehreren optischen Strukturen Licht bei der Wellenlänge reflektieren, die sich von der definierten Wellenlänge unterscheidet.

9. Haarschneidevorrichtung nach Anspruch 8, wobei der Prozessor konfiguriert ist, um:
ein erstes Befehlssignal zu erzeugen, wenn die bestimmte Beschaffenheit des Objekts anzeigt, dass das Objekt ein Haar ist, wobei das erste Befehlssignal einen Befehl an die erste Lichtquelle umfasst, ein relativ leistungsstarkes Laserlicht zu erzeugen.

10. Haarschneidevorrichtung nach Anspruch 8 oder 9, wobei der Prozessor konfiguriert ist, um:
ein zweites Befehlssignal zu erzeugen, wenn die bestimmte Beschaffenheit des Objekts anzeigt, dass das Objekt Haut oder eine Hautläsion ist, wobei das zweite Befehlssignal einen Befehl an die erste Lichtquelle umfasst, ein relativ leistungsschwaches Laserlicht zu erzeugen.

11. Verfahren zum Betreiben einer Haarschneidevorrichtung, wobei die Haarschneidevorrichtung eine erste Lichtquelle zum Erzeugen von Laserlicht, eine zweite Lichtquelle zum Erzeugen von Breitbandlicht und ein mit der ersten Lichtquelle und der zweiten Lichtquelle gekoppeltes Schneidelement umfasst, wobei das Schneidelement einen Lichtwellenleiter mit einer Vielzahl von entlang seiner Länge beabstandeten optischen Strukturen umfasst, wobei das Verfahren umfasst:
Abgabe von Breitbandlicht entlang des Lichtwellenleiters;
Erfassen einer Reaktion von Breitbandlicht, das von jeder der Vielzahl von optischen Strukturen reflektiert wird, wobei jede Reaktion eine repräsentative Frequenz aufweist; und
bei Erfassen einer spezifischen Reaktion mit einer spezifischen Frequenz, die sich von der repräsentativen Frequenz unterscheidet, Erzeugen eines Befehlssignals.

12. Verfahren nach Anspruch 11, umfassend:
Bestimmen, anhand der spezifischen Reaktion, der Beschaffenheit eines Objekts, das wahrscheinlich mit dem Lichtwellenleiter in Kontakt gekommen ist, um die Reaktion der spezifischen Frequenz zu bewirken.

13. Verfahren nach Anspruch 12, wobei, wenn bestimmt wird, dass das Objekt, das wahrscheinlich mit dem Lichtwellenleiter in Kontakt gekommen ist, ein Haar ist, dann das erzeugte Befehlssignal einen Befehl an die erste Lichtquelle umfasst, ein relativ leistungsstarkes Laserlicht zu erzeugen.

14. Verfahren nach Anspruch 12 oder 13, wobei, wenn bestimmt wird, dass das Objekt, das wahrscheinlich mit dem Lichtwellenleiter in Kontakt gekommen ist, Haut oder eine Hautläsion ist, dann das erzeugte Befehlssignal einen Befehl an die erste Lichtquelle umfasst, ein relativ leistungsarmes Laserlicht zu erzeugen.

## Revendications

1. Élément de coupe à utiliser dans un dispositif de coupe de cheveux, l'élément de coupe comprenant :
un guide d'onde optique ayant une paroi latérale, dans lequel une partie de la paroi latérale forme une face de coupe pour entrer en contact avec les cheveux, dans lequel le guide d'onde optique inclut une pluralité de structures optiques espacées sur sa longueur, les structures optiques étant configurées pour réfléchir la lumière à une ou plusieurs longueurs d'onde.
dans lequel le guide d'onde optique est tel que, lorsque de la lumière à large bande est dirigée le long du guide d'onde optique :
- si la face de coupe du guide d'onde optique n'est pas en contact avec un objet cible, alors chacune de la pluralité de structures optiques réfléchit la lumière ayant une première longueur d'onde ; et
- si la face de coupe du guide d'onde optique est en contact avec un objet cible, alors au moins l'une des structures optiques réfléchit la lumière ayant une seconde longueur d'onde différente de la première longueur d'onde.

2. Élément de coupe selon la revendication 1, dans lequel chacune de la pluralité de structures optiques comprend un réseau de Bragg à fibres ou un cristal photonique.

3. Élément de coupe selon la revendication 1 ou la revendication 2, dans lequel chacune des structures optiques est configurée pour réfléchir la lumière ayant une longueur d'onde unique.

4. Élément de coupe selon l'une quelconque des revendications 1 à 3, dans lequel le guide d'onde optique est tel que, lorsque de la lumière ayant une longueur d'onde spécifique est dirigée le long du guide d'onde optique, une partie de la lumière ayant la longueur d'onde spécifique est capable de se coupler depuis la face de coupe du guide d'onde optique dans les cheveux pour initier la coupe ou la fusion des cheveux.

5. Dispositif de coupe de cheveux pour couper les cheveux sur un corps d'un sujet, le dispositif de coupe de cheveux comprenant :
une première source de lumière pour générer de la lumière laser à une ou plusieurs longueurs d'onde spécifiques correspondant à des longueurs d'onde absorbées par un ou plusieurs chromophores dans les cheveux ;
une seconde source de lumière pour générer de la lumière à large bande ; et
un élément de coupe couplé à la première source de lumière pour recevoir de la lumière laser et à la seconde source de lumière pour recevoir de la lumière à large bande, l'élément de coupe comprenant un élément de coupe selon l'une quelconque des revendications 1 à 4.

6. Dispositif de coupe de cheveux selon la revendication 5, comprenant en outre :
un détecteur pour détecter de la lumière réfléchie depuis une ou plusieurs de la pluralité de structures optiques.

7. Dispositif de coupe de cheveux selon la revendication 5 ou la revendication 6, comprenant en outre un processeur configuré pour :
déterminer si une partie de la lumière réfléchie depuis une ou plusieurs de la pluralité de structures optiques a une longueur d'onde différente d'une longueur d'onde définie ; et
lorsque l'on détermine que la longueur d'onde de la lumière réfléchie est différente d'une longueur d'onde définie, générer un signal d'instruction.

8. Dispositif de coupe de cheveux selon la revendication 7, dans lequel le processeur est configuré pour :
déterminer, sur la base de la longueur d'onde de la lumière réfléchie, la nature d'un objet susceptible d'être entré en contact avec le guide d'onde optique, amenant les une ou plusieurs structures optiques à réfléchir la lumière à la longueur d'onde différente de la longueur d'onde définie.

9. Dispositif de coupe de cheveux selon la revendication 8, dans lequel le processeur est configuré pour :
générer un premier signal d'instruction quand la nature déterminée de l'objet indique que l'objet est un cheveu, dans lequel le premier signal d'instruction comprend une instruction à l'attention de la première source de lumière pour générer de la lumière laser à puissance relativement élevée.

10. Dispositif de coupe de cheveux selon la revendication 8 ou la revendication 9, dans lequel le processeur est configuré pour :
générer un second signal d'instruction quand la nature déterminée de l'objet indique que l'objet est de la peau ou une lésion de la peau, dans lequel le second signal d'instruction comprend une instruction à l'attention de la première source de lumière pour générer de la lumière laser à puissance relativement faible.

11. Procédé de fonctionnement d'un dispositif de coupe de cheveux, le dispositif de coupe de cheveux comprenant une première source de lumière pour générer de la lumière laser, une seconde source de lumière pour générer de la lumière à large bande, et un élément de coupe couplé à la première source de lumière et à la seconde source de lumière, l'élément de coupe comprenant un guide d'onde optique ayant une pluralité de structures optiques espacées sur sa longueur, le procédé comprenant :
la délivrance de lumière à large bande le long du guide d'onde optique ;
la détection d'une réponse à partir de la lumière à large bande réfléchie par chacune de la pluralité de structures optiques, chaque réponse ayant une fréquence représentative ; et
lors de la détection d'une réponse particulière ayant une fréquence particulière différente de la fréquence représentative, la génération d'un signal d'instruction.

12. Procédé selon la revendication 11, comprenant :
la détermination, à partir de la réponse particulière, de la nature d'un objet susceptible d'avoir été en contact avec le guide d'onde optique pour provoquer la réponse de la fréquence particulière.

13. Procédé selon la revendication 12, dans lequel, si l'on détermine que l'objet susceptible d'avoir été en contact avec le guide d'onde optique est un cheveu, alors le signal d'instruction généré comprend une instruction à l'attention de la première source de lumière pour générer de la lumière laser à puissance relativement élevée.

14. Procédé selon la revendication 12 ou revendication 13, dans lequel, si l'on détermine que l'objet susceptible d'avoir été en contact avec le guide d'onde optique est de la peau ou une lésion de la peau, alors le signal d'instruction généré comprend une instruction à l'attention de la première source de lumière pour générer de la lumière laser à puissance relativement faible.
